# EUROPÄISCHE PATENTSCHRIFT

(11) **EP 3 182 107 B1**
(45) Veröffentlichungstag und Bekanntmachung des Hinweises auf die Patenterteilung: **31.07.2019**
(21) Anmeldenummer: 16194510.0
(22) Anmeldetag: 19.10.2016
(51) Int. Cl.: G01N 27/08

(54) **MESSVORRICHTUNG UND VERFAHREN ZUR MESSUNG DER ELEKTRISCHEN LEITFÄHIGKEIT EINES STRÖMENDEN FLUIDS**
MEASURING DEVICE AND METHOD FOR MEASURING THE ELECTRICAL CONDUCTIVITY OF A FLOWING FLUID
DISPOSITIF DE MESURE ET PROCÉDÉ DE MESURE DE LA CONDUCTIVITÉ ÉLECTRIQUE D'UN FLUIDE EN CIRCULATION

(30) Priorität: 16.12.2015 DE 102015225502
(43) Veröffentlichungstag der Anmeldung: 21.06.2017
(73) Patentinhaber: Achenbach Buschhütten GmbH & Co. KG, 57223 Kreuztal (DE)
(72) Erfinder: Barten, Axel, 57223 Kreuztal (DE); Latzel, Werner, 77704 Oberkirch (DE)
(74) Vertreter: advotec.

(56) Entgegenhaltungen:
- EP-B1- 0 819 938
- US-A- 3 993 945
- MICHAEL KUHNLEIN ET AL: "Electrostatic charge measurement in hydraulic circuits", PROCEEDINGS OF THE 48TH SCANDINAVIAN CONFERENCE ON SIMULATION AND MODELING (SIMS 2007), Bd. 92, 9. September 2013 (2013-09-09), Seiten 273-283, XP055362455, Sweden (Goteborg) ISSN: 1650-3686, DOI: 10.3384/ecp1392a27 ISBN: 978-91-7685-817-2

## Beschreibung

Die Erfindung betrifft eine Messvorrichtung zur Messung der elektrischen Leitfähigkeit eines strömenden Fluids, bestehend aus einem Basissensor mit einer Messelektronik, wobei der Basissensor eine als elektrisch leitender Rohrabschnitt ausgebildete mit einem Erdpotential verbundene Außenelektrode aufweist, in der eine von dem Fluid umströmte Innenelektrode angeordnet ist, die eine Nutzelektrode aufweist, die zur Messsignalerfassung mit einem Messpotential verbunden ist, und die mindestens eine von der Nutzelektrode isoliert angeordnete Schirmelektrode umfasst, die zur Erzeugung einer geeigneten elektrischen Feldverteilung mit einem dem Messpotential entsprechenden, aber von dem Messpotential unabhängigen, Schirmpotential verbunden ist.

Weiterhin betrifft die Erfindung ein Verfahren zur Messung der elektrischen Leitfähigkeit eines strömenden Fluids mit einer erfindungsgemäßen Messvorrichtung.

Um während des Walzprozesses von metallischen flächigen Gütern, wie beispielsweise dem Auswalzen von Stahl-, Aluminium- oder Magnesiumblechen, der Gefahr von elektrostatischen Entladungsvorgängen und der damit verbundenen Brandgefahr in Walzwerken entgegenzuwirken, sollte die elektrische Leitfähigkeit des Walzöls laufend überprüft werden. Bei Unterschreitung eines bestimmten Leitfähigkeitswertes kann es daher erforderlich sein, dem Walzöl Mittel zur Erhöhung der Leitfähigkeit, sogenannte Leitfähigkeitsadditive, beizumischen.

Auf dem Markt sind Messgeräte erhältlich, mit denen nach verschiedenen Verfahren die elektrische Leitfähigkeit von Fluiden bestimmt werden kann. Nur wenige Leitfähigkeitsmessgeräte für nicht leitende oder gering leitende Fluide wie beispielsweise (Walz-)Öle oder Kraftstoffe sind jedoch zur Messung in strömenden Medien (Online-Betrieb) geeignet. Das zu messende Fluid muss sich bei der Mehrzahl der bekannten Messsysteme mehrere Minuten im Ruhezustand befinden, damit das Messgerät einen verlässlichen Wert anzeigt. Die laufende Entnahme und separate Auswertung von Messproben (Offline-Betrieb) ist zeit- und arbeitsaufwendig und mit entsprechenden Kosten verbunden. Ein weiterer Nachteil der Offline-Messgeräte ist ferner darin zu sehen, dass nur begrenzt verlässliche Messwerte der elektrischen Leitfähigkeit bestimmt werden können, da sich die Leitfähigkeit binnen kurzer Zeit durch äußere Einflüsse ändern kann. Da zwischen Probeentnahme und Messzeitpunkt, bedingt durch die von dem Messgerätehersteller vorgeschriebene Ruhezeit des Fluids, nennenswerte Zeitspannen liegen, kann es unter Umständen zur Beseitigung der Gefahr elektrostatischer Entladungen bereits zu spät sein.

Dies gilt in besonderem Maße für einen mechanischen Filtervorgang innerhalb eines Walzprozesses, bei dem aus dem Walzöl der Abrieb des gewalzten Materials herausgefiltert wird und durch das Zerteilen und Verästeln des Ölstromes über die Poren und feinen Kapillaren eines Filtermittels wie Kieselgur große elektrische Ladungsmengen durch Ladungstrennung oder Reibungselektrizität erzeugt werden.

Ein weiterer entscheidender Nachteil der auf dem Markt befindlichen Messgeräte zur Messung der elektrischen Leitfähigkeit von nicht leitenden oder gering leitenden Fluiden ist darin zu sehen, dass unvermeidbare parasitäre elektrische Kapazitäten der in den Messgeräten verwendeten Sensoren, ebenso wie Polarisationskapazitäten, große Messabweichungen bewirken.

Die fehlerbehaftete Funktionsweise der auf dem Markt befindlichen Geräte zur Messung der elektrischen Leitfähigkeit von nicht leitenden oder gering leitenden Fluiden führt dazu, dass den Fluiden, wie beispielsweise dem Walzöl, Mittel zur Erhöhung der elektrischen Leitfähigkeit vorbeugend beigegeben werden oder dass bei der Filterung von Ölen Filtermittel wie Kieselgur und Bleicherde unnötigerweise weit vor oder zu spät erst nach ihrer Erschöpfung ausgetauscht werden.

Des Weiteren sind bekannte als Leitfähigkeitsadditive oftmals ökologisch und gesundheitlich bedenklich, sodass eine unkontrollierte, vorbeugende Beimischung dieser Mittel hohe Umweltbelastungen und entsorgungstechnische Schwierigkeiten verursacht. Durch eine aufgrund eines verstärkten Einsatzes von Filtermitteln bewirkte hohe Filtratqualität und durch Reaktionen der Leitfähigkeitsadditive mit den Filtermitteln kann die Leitfähigkeit des Öles und der Ölerzeugnisse zudem stark vermindert werden. Infolge der verminderten Leitfähigkeit entstehen wiederum hohe elektrische Aufladungen, die zu unkontrollierten Verpuffungen und dem Brand von Maschinen und Anlagen führen können.

Aus diesen Betrachtungen folgt, dass eine kontrollierte Einleitung von Leitfähigkeitsadditiven und die damit einhergehende Messung der elektrischen Leitfähigkeit des nicht leitenden oder gering leitenden Fluids notwendig sind.

Aus der Patentschrift EP 0 819 938 B1 sind ein Verfahren und ein Messsystem bekannt, die eine Messung der elektrischen Leitfähigkeit eines strömenden Fluids zur Regelung einer elektrischen Mindestleitfähigkeit des Fluids beschreiben, um elektrostatische Auf- und Entladungen durch eine geregelte Einleitung von Leitfähigkeitsadditiven zu verhindern. Dabei weist das Messsystem einen Basissensor auf, der im Wesentlichen aus einer rohrförmigen Außenelektrode besteht, in die eine aus einer Nutzelektrode und Schirmelektroden bestehende Innenelektrode eingeführt ist, die von dem Fluid umspült wird. Eine Messelektronik mit einer analogen Rechenschaltung, die im Wesentlichen aus einem Umkehrintegrator und einer Triggerschaltung besteht, beaufschlagt die als Sensorfläche dienende Nutzelektrode mit einer bipolar getakteten Gleichspannung als Messsignal. Dabei stellt sich eine Grundfrequenz der bipolar getakteten Gleichspannung ein, die linear abhängig von der elektrischen Leitfähigkeit des Fluids ist.

Auf die Schirmelektroden wird dabei eine dem Messsignal entsprechende, aber von dem Messpotential unabhängige Messspannung (Potentialdifferenz) geschaltet, deren Massepotential dem Massepotential der Messelektronik ("aktive Masse") entspricht.

Allerdings erweist sich bei dieser Anordnung als nachteilig, dass bei Auftreffen des Fluids auf die Schirmelektrode die gesamte elektrische Ladung an die Messelektronik abgeführt und verarbeitet werden muss, da die Schirmelektrode mit dem aktiven Massepotential der Messelektronik elektrisch leitend verbunden ist. Bei sehr geringer Leitfähigkeit des die Innenelektrode umspülenden Fluids sind hohe elektrostatische Aufladungspotentiale und damit hohe Ableitströme zu erwarten.

Insbesondere bei elektrischen Anlagen in explosionsgefährdeten Bereichen (Ex-Bereiche) sind jedoch nur geringe Stromflüsse zugelassen. Da zudem die Ableitung der Ladungsenergie nicht niederohmig genug erfolgt, besteht die Gefahr von Signaleinbrüchen und daraus resultierenden Fehlmessungen.

Aus diesem Grund weisen für explosionsgefährdete Bereiche vorgesehene Messanordnungen oftmals miniaturisierte Elektrodenflächen auf. Diese Maßnahme reduziert zwar die Ableitungsströme, verschlechtert jedoch das Signal-Rausch-Verhältnis des Messsignals, verringert insgesamt die Messgenauigkeit und verkürzt die Betriebsdauer zwischen Servicezyklen.

Um die Zuverlässigkeit und die Genauigkeit der Messung zu gewährleisten, ist eine einwandfreie Isolation zwischen den Elektroden erforderlich. Ein Sinken eines Isolationswiderstands zwischen den Elektroden, beispielsweise infolge von Ablagerungen leitfähiger Stoffe, hätte nachteilige Auswirkungen auf das Messergebnis und würde verkürzte Reinigungsintervalle nach sich ziehen.

Der vorliegenden Erfindung liegt somit die Aufgabe zu Grunde, insbesondere in explosionsgefährdeten Bereichen die Messung der elektrischen Leitfähigkeit eines strömenden Fluids elektrisch sicher und zuverlässig zu gestalten.

Diese Aufgabe wird bezogen auf eine Vorrichtung in Verbindung mit dem Oberbegriff des Anspruchs 1 dadurch gelöst, dass der Basissensor zur Ableitung elektrischer Ladungen mindestens eine von dem Fluid umströmte Entladeelektrode aufweist, die mit der Außenelektrode elektrisch leitend verbunden ist.

Erfindungsgemäß weist der Basissensor mindestens eine Entladeelektrode auf, die unmittelbar mit der auf Erdpotential liegenden Außenelektrode widerstandsfrei elektrisch leitend verbunden ist. Dadurch werden die elektrischen Ladungen niederohmig, direkt ohne Umweg über die Messelektronik, abgeleitet. Die die elektrische Sicherheit gefährdenden elektrostatischen Potentiale werden abgebaut und das Gefahrenpotential für die Entstehung eines Zündfunkens wird deutlich reduziert.

In weiterer Ausgestaltung ist zwischen der Nutzelelektrode und der Schirmelektrode sowie zwischen der Schirmelektrode und der Entladeelektrode jeweils eine Isolierhülse angeordnet, die mit einer wasserabweisenden und/oder einer ölabweisenden Beschichtung versehen ist.

Um ein Sinken des Isolationswiderstands zwischen den Elektroden durch Anbackungen oder Anlagerungen, beispielsweise von Leitfähigkeitsadditiven, zu vermeiden, ist zwischen benachbarten Elektroden eine Isolierhülse angeordnet, die eine wasserabweisende und/oder eine ölabweisende Beschichtung aufweist. Die Beschichtung verhindert weitgehend die Anlagerung leitfähiger Substanzen und stellt damit eine hohe Messqualität in Form eines unverfälschten Messergebnisses bei geringem Messstrom über eine lange Betriebsdauer sicher.

In vorteilhafter Weise besteht die wasserabweisende und/oder ölabweisende Beschichtung aus Partikeln im Nanomaßstab.

Mit nanoskaligen Partikeln lassen sich die Eigenschaften einer wasserabweisenden und/oder ölabweisenden Oberfläche der Isolierhülsen gezielt herstellen.

Bezogen auf ein der Erfindung zugrunde liegendes Verfahren wird die Aufgabe in Verbindung mit dem Oberbegriff des Anspruchs 4 dadurch gelöst, dass eine unmittelbare Ableitung elektrischer Ladungen mittels mindestens einer Entladeelektrode erfolgt, die über die Außenelektrode elektrisch leitend mit dem Erdpotential verbunden ist.

Aufbauend auf dem aus der Patentschrift EP 0 819 938 B1 bekannten Messprinzip werden das dort offenbarte Messsystem und das diesem Messsystem zugrundeliegende Verfahren dahingehend modifiziert, dass eine unmittelbare Ableitung elektrischer Ladungen mittels einer Entladeelektrode erfolgt, die über die Außenelektrode widerstandsfrei elektrisch leitend mit dem Erdpotential verbunden ist.

Da die Messelektronik somit nur noch die während des Messvorgangs aus der Beaufschlagung mit der bipolar getakteten Gleichspannung hervorgerufenen (Umlade-)Ströme verarbeiten muss und die für die Messung erforderliche Stromzufuhr hochohmiger ausgelegt werden kann, wird der in explosionsgefährdeten Bereichen kritische Gesamtstrombedarf der Messelektronik auf ein Minimum beschränkt. Dies ermöglicht eine sichere und zuverlässige Messung der elektrischen Leitfähigkeit des strömenden Fluids.

Die Messelektronik umfasst im Wesentlichen eine in analoger und/oder digitaler Schaltungstechnik realisierte Integrator- und Kippschaltungsfunktion, die die erfassten elektrischen Messgrößen in eine Frequenz transformiert.

Die Messelektronik ist dabei so ausgelegt, dass sich in Abhängigkeit des zwischen der Nutzelektrode und der Außenelektrode wirkenden elektrischen Widerstands des Fluids und damit in linearer Abhängigkeit von der elektrischen Leitfähigkeit des Fluids eine Grundfrequenz der bipolar getakteten Gleichspannung einstellt.

Diese Frequenz bildet somit ein Maß für die elektrische Leitfähigkeit des Fluids. Sie ist leicht messbar und kann in einfacher Weise digitalisiert und störungsfrei fernübertragen werden.

Weitere vorteilhafte Ausgestaltungsmerkmale ergeben sich aus der nachfolgenden Beschreibung und den Zeichnungen, die eine bevorzugte Ausführungsform der Erfindung an Hand eines Beispiels erläutern. Es zeigen:
- **Fig. 1:**: einen Basissensor nach dem Stand der Technik und
- **Fig. 2:**: einen erfindungsgemäßen Basissensor mit Entladeelektroden.

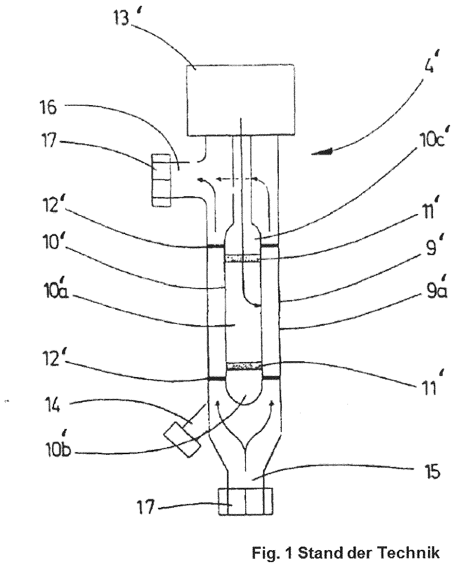

In **Fig. 1** ist ein aus der Patentschrift EP 0 819 938 B1 bekannter Basissensor 4' dargestellt.

Der Basissensor 4' weist eine Außenelektrode 9' auf, die als ein elektrisch leitender metallischer Rohrabschnitt 9a' ausgebildet ist, in dem eine elektrisch leitende, vom Fluid umströmte, vorzugsweise stromlinienförmig ausgebildete Innenelektrode 10' angeordnet ist. Die zylindrische Innenelektrode 10' ist in eine zentral angeordnete Nutzelektrode 10a' sowie eine - bezogen auf die Strömungsrichtung des Fluids - vordere und eine hintere Schirmelektrode 10b', 10c' aufgeteilt. Die Nutzelektrode 10a' der Innenelektrode 10' ist durch Isolierhülsen 11' von den beiden Schirmelektroden 10b', 10c' getrennt. Die Innenelektrode 10' ist durch zwei Distanzhalter 12' in dem Basissensor 4' gehalten, wobei die Distanzhalter 12' zwischen der Außenelektrode 9' und den beiden Schirmelektroden 10b', 10c' eingebaut sind.

Durch diese Anordnung werden inhomogene Randfelder vermieden, die bei einer Ablagerung von elektrisch leitenden Schmutzpartikeln an den Distanzhaltern 12' zu falschen Messergebnissen führen können. Die Innenelektrode 10' ist zur Durchführung von Anschlussleitungen für eine Messelektronik 13' teilweise als Hohlkörper ausgebildet.

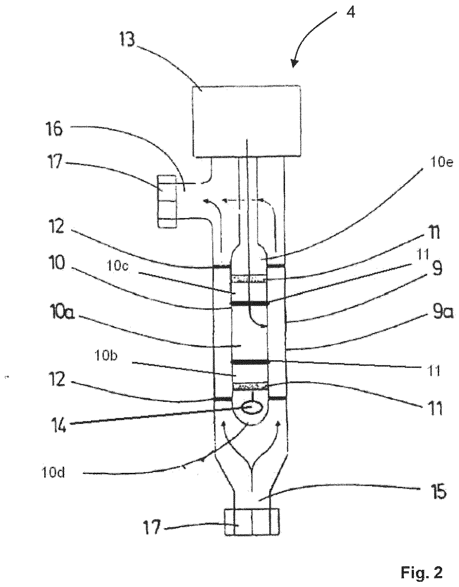

Die **Fig.2** zeigt einen erfindungsgemäßen Basissensor 4. Dieser weist in der dargestellten beispielhaften Ausführungsform eine koaxiale Anordnung aus einer Innenelektrode 10 auf, die von einer zylinderförmigen Außenelektrode 9, 9a umschlossen wird.

Die Innenelektrode 10 des erfindungsgemäßen Basissensors 4 besteht aus einer zentral angeordneten Nutzelektrode 10a, an die sich eine vordere und eine hintere Schirmelektrode 10b, 10c anschließen, die jeweils durch Isolierhülsen 11 von der Nutzelektrode 10a elektrisch getrennt sind.

Die als Sensorfläche dienende Nutzelektrode 10a wird von einer Messelektronik 13 mit einer bipolar getakteten Gleichspannung als Messsignal beaufschlagt.

Auf die Schirmelektroden 10b, 10c wird eine dem Messsignal entsprechende, aber von dem Messpotential unabhängige Messspannung geschaltet, deren Massepotential dem aktiven Massepotential der Messelektronik entspricht. Durch die Anordnung der aktiven Schirmflächen entsteht im Bereich der Nutzelektrode eine gleichförmige, radial gerichtete elektrostatische Feldverteilung, so dass es bei der Bestimmung des elektrischen Widerstands des zwischen Nutz- und Außenelektrode 10a, 9 vorbeiströmenden Fluids nicht erforderlich ist, Randeffekte an der Sensorfläche der Nutzelektrode 10a zu berücksichtigen.

In Erweiterung des aus dem Stand der Technik bekannten Basissensors 4' umfasst der erfindungsgemäße Basissensor 4 zusätzlich zwei Entladeelektroden 10d, 10e, die jeweils vor und hinter den Schirmelektroden 10b, 10c angeordnet und von diesen durch weitere Isolierhülsen 11 elektrisch getrennt sind. Die Entladeelektroden 10d, 10e sind unmittelbar mit der auf Erdpotential liegenden Außenelektrode 9 elektrisch leitend verbunden und gewährleisten eine direkte Ableitung elektrischer Ladungen ohne die Messelektronik 13 durch Ableitströme zu belasten. Damit kann der Gesamtstrombedarf der Messelektronik 13 gesenkt werden, wobei die Messgenauigkeit auch bei dieser limitierten Stromzufuhr erhalten bleibt.

Zur Bestimmung der elektrischen Leitfähigkeit des zwischen der Nutzelektrode 10a und der zylinderförmigen Außenelektrode 9, 9a vorbeiströmenden Fluids, hier beispielhaft des Walzöls, wird der ohmsche Widerstand des Öls zwischen der zylinderförmigen Außenelektrode 9, 9a und der Nutzelektrode 10a mit der Messelektronik 13 ausgewertet, wobei sich eine Grundfrequenz der angelegten bipolar getakteten Gleichspannung ergibt, die linear abhängig von der elektrischen Leitfähigkeit des Walzöls ist.

Zur Lagerung der Anordnung aus Innenelektrode 10 mit Nutz- und Schirmelektroden 10a, 10b, 10c und Endladeelektroden 10d, 10e in dem Basissensor 4 sind die Entladeelektroden 10d, 10e mit zwei Distanzhaltern 12 versehen, die die innenliegende Anordnung gegen die Außenelektrode 9 abstützen.

Ein in eine Entladeelektrode 10d des Basissensors 4 eingebauter Temperatursensor 14 dient zur Messung der Temperatur des Walzöls.

Die zylinderförmige Außenelektrode 9, 9a weist einen Eintritts- und einen Austrittsstutzen 15, 16 jeweils mit einem Rohranschluss 17 für den Einbau des Basissensors 4 in den Kreislauf des Fluids (Walzölkreislauf) auf.

## Patentansprüche

1. Messvorrichtung zur Messung der elektrischen Leitfähigkeit eines strömenden Fluids, bestehend aus einem Basissensor (4) mit einer Messelektronik (13), wobei der Basissensor (4) eine als elektrisch leitender Rohrabschnitt (9a) ausgebildete mit einem Erdpotential verbundene Außenelektrode (9) aufweist, in der eine von dem Fluid umströmte Innenelektrode (10) angeordnet ist, die eine Nutzelektrode (10a) aufweist, die zur Messsignalerfassung mit einem Messpotential verbunden ist, wobei die Messelektronik (13) eine in analoger und/oder digitaler Schaltungstechnik realisierte Integrator- und Kippschaltungsfunktion aufweist, die so ausgelegt ist, dass sich bei einer als Messpotential angelegten bipolar getakteten Gleichspannung in linearer Abhängigkeit von der elektrischen Leitfähigkeit des Fluids eine Grundfrequenz der bipolar getakteten Gleichspannung einstellt, und wobei die Innenelektrode (10) mindestens eine von der Nutzelektrode (10a) isoliert angeordnete Schirmelektrode (10b, 10c) umfasst, die zur Erzeugung einer geeigneten elektrischen Feldverteilung mit einem dem Messpotential entsprechenden, aber von dem Messpotential unabhängigen, Schirmpotential verbunden ist, sodass an der Schirmelektrode (10b, 10c) eine der bipolaren getakteten Gleichspannung entsprechende Spannung geschaltet wird,
**dadurch gekennzeichnet,**
**dass** der Basissensor (4) zur Ableitung elektrischer Ladungen mindestens eine von dem Fluid umströmte Entladeelektrode (10d, 10e) aufweist, die mit der Außenelektrode (9) elektrisch leitend verbunden ist.

2. Messvorrichtung nach Anspruch 1
**dadurch gekennzeichnet,**
**dass** zwischen der Nutzelelektrode (10a) und der Schirmelektrode (10b, 10c) sowie zwischen der Schirmelektrode (10b, 10c) und der Entladeelektrode (10d, 10e) jeweils eine Isolierhülse (11) angeordnet ist, die mit einer wasserabweisenden und/oder einer ölabweisenden Beschichtung versehen ist.

3. Messvorrichtung nach Anspruch 2
**dadurch gekennzeichnet,**
**dass** die wasserabweisende und/oder ölabweisende Beschichtung aus Partikeln im Nanomaßstab besteht.

4. Verfahren zur Messung der elektrischen Leitfähigkeit eines strömenden Fluids mit einer Messvorrichtung nach einem der Ansprüche 1 bis 3, wobei
eine bipolar getaktete Gleichspannung als Messsignal an die Nutzelektrode angelegt wird und sich eine Grundfrequenz der bipolar getakteten Gleichspannung in linearer Abhängigkeit von der Leitfähigkeit des Fluids einstellt,
**dadurch gekennzeichnet, dass**
eine unmittelbare Ableitung elektrischer Ladungen mittels mindestens einer Entladeelektrode erfolgt, die über die Außenelektrode elektrisch leitend mit dem Erdpotential verbunden ist.

## Claims

1. A measuring device for measuring the electrical conductivity of a flowing fluid, composed of a basic sensor (4) having measuring electronics (13), the basic sensor (4) having an outer electrode (9) which is configured as an electrically conductive tube section (9a) and connected to a ground potential and in which an inner electrode (10) is disposed, the inner electrode (10) being surrounded by the flowing fluid and having a useful electrode (10a) which is connected to a measuring potential for measuring-signal detection, the measuring electronics (13) having an integrator and rocker-switch function which is realized by analog and/or digital circuit technology and which is configured in such a manner that when a bipolar clocked direct voltage is applied as a measuring potential, a basic frequency of the bipolar clocked DC voltage is generated as a linear function of the electrical conductivity of the fluid, and the inner electrode (10) comprising at least one shield electrode (10b, 10c) which is disposed in isolation from the useful electrode (10a) and connected to a shield potential corresponding to the measuring potential but independent therefrom in order to generate a suitable electric field distribution so that a voltage corresponding to the bipolar clocked DC voltage is switched to the shield electrode (10b, 10c),
**characterized in that**
for discharging electrical charges, the basic sensor (4) has at least one discharge electrode (10d, 10e) around which the fluid flows and which is connected to the outer electrode (9) in an electrically conductive manner.

2. The measuring device according to claim 1,
**characterized in that**
insulating sleeves (11) provided with a water-repellent and/or oil-repellent coating are disposed between the useful electrode (10a) and the shield electrode (10b,10c) and between the shield electrode (10b, 10c) and the discharge electrode (10a, 10e).

3. The measuring device according to claim 2,
**characterized in that**
the water-repellent and/or oil-repellent coating is made of nanoscale particles.

4. A method for measuring the electrical conductivity of a flowing fluid using a measuring device according to any one of claims 1 to 3, wherein
a bipolar clocked DC voltage is applied to the useful electrode as a measuring signal and a basic frequency of the bipolar clocked DC voltage is generated as a linear function of the conductivity of the fluid,
**characterized in that**
electrical charges are immediately discharged by means of at least one discharge electrode which is connected to the ground potential in an electrically conductive manner via the outer electrode.

## Revendications

1. Dispositif de mesure de la conductivité électrique d'un fluide coulant, composé d'un capteur de base (4) ayant un système électronique de mesure (13), le capteur de base (4) ayant une électrode extérieure (9) qui est réalisée comme tronçon tubulaire (9a) électriquement conducteur et connectée à un potentiel de terre et dans laquelle une électrode intérieure (10) entourée par le fluide coulant est disposée, l'électrode intérieure (10) comprenant une électrode utile (10a) qui est connectée à un potentiel de mesure afin de détecter des signaux de mesure, le système électronique de mesure (13) ayant une fonction d'intégrateur et d'interrupteur à bascule qui est réalisée en technologie de circuit analogique et/ou numérique de telle manière que lorsqu'une tension continue cadencée bipolaire est appliquée comme potentiel de mesure, une fréquence de base de la tension continue cadencée bipolaire se présente en fonction linéaire de la conductivité électrique du fluide, et l'électrode intérieure (10) comprenant au moins une électrode de blindage (10b, 10c) qui est disposée de manière isolée de l'électrode utile (10a) et qui est connectée à un potentiel de blindage correspondant au potentiel de mesure mais indépendant du potentiel de mesure afin de générer une distribution appropriée du champ électrique de sorte qu'une tension correspondant à la tension continue cadencée bipolaire est couplée à l'électrode de blindage (10b, 10c),
**caractérisé en ce que**
le capteur de base (4) a au moins une électrode de décharge (10d, 10e) pour décharger des charges électriques qui est entourée par le fluide coulant et connectée de manière électriquement conductrice à l'électrode extérieure (9).

2. Dispositif de mesure selon la revendication 1,
**caractérisé en ce**
**qu'**une gaine isolante (11) est disposée entre l'électrode utile (10a) et l'électrode de blindage (10b, 10c) et entre l'électrode de blindage (10b, 10c) et l'électrode de décharge (10d, 10e), chaque gaine isolante (11) étant munie d'un revêtement hydrofuge et/ou oléofuge.

3. Dispositif de mesure selon la revendication 2,
**caractérisé en ce que**
le revêtement hydrofuge et/ou oléofuge se compose de particules à l'échelle nanométrique.

4. Procédé de mesure de la conductivité électrique d'un fluide coulant en utilisant un dispositif de mesure selon l'une quelconque des revendications 1 à 3, dans lequel
une tension continue cadencée bipolaire est appliquée à l'électrode utile comme signal de mesure et une fréquence de base de la tension continue cadencée bipolaire se présente en fonction linéaire de la conductivité du fluide,
**caractérisé en ce que**
des charges électriques sont déchargées au moyen d'au moins une électrode de décharge qui est connectée de manière électriquement conductrice au potentiel de terre par l'intermédiaire de l'électrode extérieure.
